# EUROPEAN PATENT SPECIFICATION

(11) **EP 1 842 429 B1**
(45) Date of publication and mention of the grant of the patent: **12.01.2011**
(21) Application number: 05816182.9
(22) Date of filing: 07.11.2005
(51) Int. Cl.: A23D 9/007, A23L 1/30

(54) **OILY MIXTURE OF NATURAL BIOACTIVE INGREDIENTS FOR THE PREPARATION OF AN ENRICHED FOOD PRODUCT**
ÖLIGE MISCHUNG VON NATÜRLICHEN BIOLOGISCH WIRKSAMEN BESTANDTEILEN FÜR DIE HERSTELLUNG EINES ANGEREICHERTEN NAHRUNGSMITTELPRODUKTS
MELANGE HUILEUX D'INGREDIENTS BIOACTIFS NATURELS POUR LA PREPARATION D'UN PRODUIT ALIMENTAIRE ENRICHI

(30) Priority: 16.11.2004 ES 200402755
(43) Date of publication of application: 10.10.2007
(73) Proprietor: Universidad Autónoma de Madrid, 28049 Madrid (ES); Embutidos Frial, S.A., 28760 Tres Cantos, Madrid (ES)
(72) Inventor: REGLERO RADA, G., Ciudad Univ. Cantoblanco, E-28049 MADRID (ES); SEÑORANS RODRIGUEZ, J., Ciudad Univ. Cantoblanco, E-28049 MADRID (ES); IBAÑEZ EZEQUIEL, E., Ciudad Univ. Cantoblanco, E-28049 MADRID (ES); SANTOYO DIEZ, S., Ciudad Univ. Cantoblanco, E-28049 MADRID (ES); TORRES OLIVARES, C., Ciudad Univ. Cantoblanco, E-28049 MADRID (ES); JAIME DE PABLO, L., Ciudad Univ. Cantoblanco, E-28049 MADRID (ES); SOLER RIVAS, C., Ciudad Univ. Cantoblanco, E-28049 MADRID (ES); RODRIGUEZ GARCIA-RISCO, M, Ciudad Univ Cantoblanco, E-28049 MADRID (ES); MARIN MARTIN, F., Ciudad Univ. Cantoblanco, E-28049 MADRID (ES); RUIZ RODRIGUEZ, A., Ciudad Univ. Cantoblanco, E-28049 MADRID (ES); FRIAL SUAREZ, P., E-28760 TRES CANTOS (Madrid) (ES)
(74) Representative: Carpintero Lopez, Francisco
(86) International application number: PCT/ES2005/000600
(87) International publication number: WO 2006/053917

(56) References cited:
- FR-A1- 2 761 887
- JP-A- 2001 346 517
- US-A- 5 077 069
- US-A1- 2003 161 918
- RINGSEIS R. ET AL.: 'Effects of Dietary Fish Oil and Oxidized Cholesterol on the Concentration of 7beta-Hydroxycholesterol in Liver, Plasma, Low Density Lipoproteins and Erythrocytes of Rats at Various Vitamin E Supply' EUR. J. LIPID SCI. TECHNOL. vol. 105, 2003, pages 121 - 129, XP003011826

## Description

### FIELD OF THE INVENTION

The present invention belongs to the field of food products enriched with natural bioactive ingredients. More specifically, it refers to an oily mixture based on natural bioactive ingredients that is added to food products, especially to meat products, in order to compensate effectively for the lipid imbalance of food products derived from land animals and to exert beneficial effects on human health, especially in the prevention of diseases, without impairing the quality or safety of these food products.

### BACKGROUND OF THE INVENTION

For several years, food products of animal origin have been appearing on the market, mainly milk products (although also some meat products), which incorporate omega-3 (ω-3) polyunsaturated fatty acids, by adding a small percentage of fish oil. Omega-3 fatty acids are not present in their natural form in meat from land animals, and their incorporation in food products of animal origin in pursuit of health benefits is based on many years of scientific experience.

Traditionally, animal fats are considered to be unhealthy. For decades, their lipid composition has been associated with the probability of suffering cardiovascular diseases. In fact, individuals with cardiovascular risk are recommended to restrict whole milk and meat products in their diets. Research has focused for many years on this aspect in an attempt to establish the scientific explanations for these occurrences. Theories for this association have developed considerably, especially in recent years. For many years, animal fat has been considered to be responsible for increased serum cholesterol levels and a direct association has been established between cholesterol levels and cardiovascular disease. More recently, triglycerides, and especially triglyceride levels in blood and the duration of these levels in the blood, have been considered to constitute the origin of the cardiovascular risk factor.

From around the middle of the last century, research has been based on establishing the effects of polyunsaturated fatty acids or PUFAs on reducing serum cholesterol levels and in cardiovascular disease. The most significant works in this area were those carried out by Ahrens et al, 1954 (Ahrens E.H., D.H. Blankenhorn, T.T. Tastas (1954), "Effect on human serum lipids of substituting plant for animal far in the diet", Proc. Soc. Exp. Biol. Med. 86, 872.) and Keys et al., 1957 (Keys A., J.T. Anderson, F. Grande (1957), "Serum cholesterol response to dietary fat", Lancet 1, 787) which established clear evidence for the importance of PUFAs in the prevention of cardiovascular diseases. Since then, numerous studies have focused on this aspect, most of which have confirmed the beneficial effects of ω-3 on the heart. For example, in a clinical trial carried out recently by researchers of the Laboratory of Cardiovascular Nutrition of the Baker Medical Research Institute of Melbourne, the Department of Medicine of the Medical Defence College of Tokio, CSIRO of the Division of Health Sciences and Nutrition of Adelaide (Australia) and Vitamin Research of F Hoffmann-La Roche (Switzerland) published in the American Journal of Clinical Nutrition (Am J Clin Nutr 76 (2002) 326-330) of the American Society for Clinical Nutrition, showed that ω-3 fatty acids, and especially long chain ones, in other words, DHA (docosahexanoic acid) and EPA (eicosapentanoic acid), can help to maintain arterial elasticity and consequently normal blood pressure levels and to reduce cardiovascular risk. The study consisted in administering DHA or EPA or placebo to patients with hypercholesterolemia for seven weeks. The researchers then measured the elasticity of the participants' arteries using ultrasound. Those who received ω-3 fatty acids presented a significant reduction in arterial sclerosis, while participants taking placebo presented no significant changes. Those taking EPA presented an increased arterial systemic resistance of 36%, reflecting the elasticity of the main arteries, while those taking DHA presented an increase of 27%.

ω-3 fatty acids (EPA/DHA) improve the blood lipid profile, since they increase the elasticity, reduce LDL cholesterol, increase HDL, reduce arterial triglyceride levels and are antithrombotic. Adeemia (López-Huertas-E; Baro,-L; Carrero,-J-J; Fonolla,-J (2003) "n-3 fatty acids: health effects and opportunities to increase intake", Agro Food Industry hi tech. 2003; 14(3): 18-21; Dewailly,-E; Blanchet,-C; Gingras,-S; Lemieux,-S; Holub,-B-J (2002), "Cardiovascular disease risk factors and n-3 fatty acid status in the adult population of James Bay Cree", American-Journal-of-Clinical-Nutrition. 2002; 76(1): 85-92).

In addition to the beneficial cardiovascular effects of ω-3, as mentioned in previous parts of the text, these fatty acids have important effects on gene expression and on other biochemical body processes. One of the most important roles of ω-3 is in the formation of cell membranes. Most brain tissues are rich in ω-3 fatty acids. Current knowledge of these effects is summarised in an article by Donald B. Jump of the Department of Physiology, Biochemistry, and Molecular Biology de Michigan State University published in the Journal of Biological Chemistry of the American Society for Biochemistry and Molecular Biology (J. Biol. Chem 227 (2002) 8755-8758)

Today it is known that polyunsaturated fatty acids (PUFA) ω-3 and ω-6 are involved in important biological processes in the human body and that the ratio of these is a key factor in the prevention of numerous chronic diseases (Simopoulos A.P. (2002), "The importance of the ratio omega-6/omega-3 essential fatty acids", Biomedicine and Pharmacotherapy 56, 365), including cancer (Nkondjock A., B. Shatenstein, P. Maisonneuve, P. Ghadirian (2003), "Specific fatty acids and human colorectal cancer: an overview", Cancer Detection and Prevention 27, 55). A recommended ratio is close to 1 (Simopoulos A.P. (1999), "Evolutionary aspects of omega-3 fatty acids in the food supli Prostaglandins, Leucotrienes and Essential Fatty Acids", 60, 421). The ω-6 / ω-3 ratio in pork meat is, on average, above 10 and in pork fat it is even higher. In both cases, therefore, it is far higher than recommended.

An interesting research work carried out by A.P. Simopoulos [Biomedicine & Pharmacotherapy 56 (2002) 365-379] described the benefits of different ω-6/ω-3 ratios. According to this work:
ω-6/ω-3= 5, has beneficial effects on asthma.
ω-6/ω-3= 4, has proven effects on reducing cardiovascular risk.
ω-6/ω-3= between 2 and 3 prevents colon cancer and rheumatoid arthritis.

The lower the ω-6/ω-3 ratio, the better its preventive effect against breast cancer.

ω-6/ω-3 higher than 10 begins to have adverse effects.

For the negative effects of ω-6 to be effectively compensated for by adding ω-3, compounds with antioxidant activity must be added simultaneously [B. Demmig-Adams y W.W. Adams, III. [Science 298 (2002) 2149-2153].

The antioxidant properties of spices has been known since the beginning of the Fifties (Chipault J.R., Muzumo G.R., Hawkins J.M., Lundberg W.O. (1952), "The antioxidant properties of natural spices", Food Res. 17, 46). In 1955 it was first discovered that rosemary was one of the herbs with the greatest antioxidant activity (Rac M., Ostric-Matijasevic B. (1955), "The properties of rosemary as an antioxidant", Rev. Fr. Corps Gras 2, 796). The compounds responsible for this have been well established. In 1966, carnosol was isolated (Briescorn C.H., Fuchs A., Bredenberg J.B., McChesney J.D., Wenkert E. (1966), "The structure of carnosol", J. Org. Chem. 29, 2293) and the plant's antioxidant properties were attributed to this phenolic diterpene. Its structure, and that of carnosic acid, were confirmed in 1982 (Wu J.W., Lee M.H., Ho C.T., Chan S.S. (1982), "Elucidation of the chemical structures of natural antioxidants isolated from Rosemary", JAOCS 59, 339) and in the same year rosmanol and rosmarinic acid were identified (Inatani R., Nakatani N., Fuwa H., Seto H. (1982), "Structure of a new antioxidative phenolic diterpene isolated from Rosemary", Agric. Biol. Chem. 46, 1666). After this, rosmadial (Inatani R., Nakatani N., Fuwa H. (1983), "Antioxidative effect of the constituents of Rosemary and their derivatives", Agric. Biol. Chem. 47, 521), epirosmanol and isorosmanol (Nakatani N., Inatani R. (1984), "Two antioxidative diterpenes from Rosemary and a revised structure for rosmanol", Agric. Biol. Chem. 48, 2081) rosmaridiphenol and rosmariquinone (Houlihan C.M., Ho C.T., Chang S.S. (1985), "The structure of rosmariquinone. A new antioxidant isolated from Rosmarinus officinalis L.", JAOCS 62, 1985) were identified. In addition to these compounds, it is known that rosemary leaves also contain flavonoids with antioxidant activity (Okamura N., Haraguchi H., Hashimoto K., Yagi A. (1994), "Flavonoids in Rosmarinus officinalis leaves", Phytochem. 37, 1463).

In general terms and considering these compounds individually, carnosic acid is found to be the one with the highest antioxidant activity, followed by carnosol, rosmarinic acid, rosmanol and rosmadial (Cuvelier M.E., Richard H., Berset C. (1996), "Antioxidative activity and phenolic composition of pilot-plant and commercial extracts of sage and rosemary", JAOCS 73, 645). Carnosol is usually the majority compound, frequently corresponding to as much as 90% of the extracts. In fact it is produced, along with other phenolic compounds found in rosemary, from the oxidation of carnosic acid during extraction operations.

Phenolic diterpenes of rosemary act as primary antioxidants (Basaga H., Tekkaya C., Acikel F. (1997), "Antioxidative and free radical scavenging properties of rosemary extract", Lebensm. Wiss. Technol. 30, 105; Frankel E.N., Shu W.H., Aeschbatch R., Prior E. (1996), "Antioxidant activity of a rosemary extract and its constituents carnosic acid, carnosol, and rosmarinic acid in bulk oil and oil-in-water emulsion", J. Agric. Food Chem. 44, 131; y Haraguchi H., Saito T., Okamura N., Yagi A. (1995), "Inhibition of lipid peroxidation and superoxide generation by diterpenoids from Rosemary officinalis", Planta Medica 61, 333). It has also been shown that these products have a similar activity to superoxide dismutase (Seok J.K., Daeseok H., Kwang D.M., Joon S.R. (1995), "Measurement of superoxide dismutase-like activity of natural antioxidants", Biosci. Biotechnol. Biochem. 59, 822) and synergic effects with the enzymes glutathione reductase and NADPH-quinone reductase, regenerating them and increasing the free radical blocking effect they have. These synergies with the enzymes mentioned have been attributed protective effects against cancerogenic agents in lung, liver and stomach, demonstrated in recent years in mice (Singletary K.W., Rokusek J.T. (1997), "Tissue specific enhancement of xenobiotic detoxification enzymes in mice by dietary rosemary extract", Plant Foods for Human Nutrition 50, 47; Offord, E.A., K. Macé, O. Avanti, A.M.A. Pfeifer (1997), "Mechanisms involved in the chemoprotective effects of rosemary extract studied in human liver and bronchial cells", Cancer Letters 114, 275). The action mechanism of antioxidants against the peroxidation of blood lipoproteins is also known, and is a key factor in the development of arteriosclerosis (Pinchuk I., D. Lichtenberg (2002), "The mechanism of action of antioxidants against lipoprotein peroxidation, evaluation based on kinetic experiments", Progress in Lipid Research 41, 279).

Rosemary is a very common ingredient in cooking. However, owing to its intense aroma and its texture, it cannot be added to meat products in sufficient quantities to have the desired effect. This is the reason for using extracts.

Supercritical extraction is a good alternative to extraction with solvents to obtain antioxidants. There are several processes to extract aromas and natural dyes, hops and oleoresins of different plants. The extraction in non-extreme and non-oxidant conditions can produce high quality products with intact natural properties free from solvent residues.

The case of oleoresins is noteworthy and these can, generally, be fractionated in the same supercritical extraction process generating products with different functions. Several applications have been carried out with extractions from labiated plants (rosemary, thyme, oregano, sage, etc.) (Nguyen U., Evans D.D., Frakman G. (1994), "Natural antioxidants produced by supercritical fluid extraction", In "Supercritical Fluid Processing of Foods and Biomaterials", Ed. S.S.H. Rizvi. Chapman & Hall, London. p.103). In these cases, using extraction with supercritical fluids or SFE (Supercritical Fluid Extraction), an oleoresin is obtained that is easily fractionable into two products: an essential oil, generally with aromatic and antimicrobial properties, and an antioxidant.

Nowadays, it is well known that natural antioxidants obtained by SFE have a greater activity than those extracted with solvents. Djarmati and coworkers (Djarmati Z., Jankov R.M., Schwirtlich E., Djulinac B., Djordjevic A. (1991), "High antioxidant activity of extracts obtained from sage by supercritical CO2 extraction", JAOCS 68, 731) showed that antioxidant extracts of sage obtained by extraction with supercritical CO₂ were more effective than BHT. More recently, the same was found to occur with extracts of black pepper. (Tipsrisukond N., Fernando L.N., Clarke A.D. (1998), "Antioxidant Effects of Essential Oil and Oleoresin of Black Pepper from Supercritical. Carbon Dioxide Extractions in Ground Pork", J. Agric. Food Chem. 46, 4329).

The phytochemical contents in rosemary antioxidant extract have important biological activities. Their effect on unsaturated fatty acids is especially interesting.

Similarly, in the state of the art, beneficial properties are known for the microalga *Dunaliella salina,* a unicellular alga belonging to the genus of green microalgae (chlorophytes). This microalga was the first one to be used commercially to produce fine chemicals since their high salinity greatly simplified culture maintenance, without danger of external contamination by pathogens (Borowitzka L.J., Moulton T.P., Borowitzka M.A. (1985), "Salinity and the commercial production of beta-carotene from Dunaliella salina", In: Barclay W.J., McIntosh R., eds. Algas Biomass: and Interdisciplinary Perspective. J. Cramer Verlag, Verduz). Currently, *Dunaliella salina* is consumed as a food supplement rich in beta-carotene (Mokady S., Abramovici A., Cogan U. (1989), "The safety evaluation of Dunaliella bardawil as a potential food supplement", Food Chem. Toxicol. 27, 221; Tanaka Y. (1990), "Process for production of encapsulated foodstuff containing Dunaliella algae", United States patent US 4,915,965, and Japanese patent JP 88-40755; Leach G., Oliveira G., Morais R. (1998), "Spray-drying of Dunaliella salina to produce a beta-carotene rich powder", J. Ind. Microb. Biotechnol. 20, 82; Orset S., Leach G.C., Morais R., Young A.J. (1999), "Spray-drying of the microalga Dunaliella salina: Effects on beta-carotene content and isomer composition", J. Agric. Food Chem. 47, 4782). Australia produces more than 80% of the beta-carotene that is consumed world-wide, all from cultures of *Dunaliella salina.* Beta-carotene is found in this microalga in concentrations of up to 14% by weight of dry weight of it, making this the alga with the highest content of this compound, the accumulation of which depends on the culture conditions (salinity, temperature, light intensity). Recent studies have achieved the isolation and purification of different beta-carotene isomers, such as 9-cis (Yamano Y., Yoshizawa M., Ito M. (1999), "Isolation of 9Z beta-carotene from Dunaliella bardawil and its stereoselective synthesis", J. Nutr. Sci. Vitamin. 45, 49) and have established its antioxidant activity compared with synthetic beta-carotene (mainly of "all-trans" composition). Other compounds present in this microalga with functional properties are the tocopherols (which are usually quantified as alpha-tocopherol since their isomeric composition is unknown), polyunsaturated fatty acids (PUFAs) (Franke H., Springer M., Pulz O., Tietz U., Mueller U. (1994), "Polyunsaturated fatty acids from microalgae", Int. Food Ingr. 4,41), sterols (such as ergosterol) and hydrosoluble vitamins (such as thiamine, pyridoxine, biotin, riboflavin, etc.). The presence of flavonoids or phenolic compounds has not been described in this alga. However, their presence would be expected since they have been detected in similar microalgae species (Rauha, JP; Remes, S; Heinonen, M; Hopia, A; Kähkönen, M; Kujala, T; Pihlaja, K; Vuorela, H; Vuorela, P. (2000), "Antimicrobial effects of Finnish plant extracts containing flavonoids and other phenolic compounds", Int. J. Food Microbiol. 56, p. 3-12).

Regarding the effect of carotenoids other than the one described previously, very recently the effects of carotenoids such as lutein have been demonstrated, for example, in the prevention of age-related macular degeneration. These effects are more pronounced when these carotenoids are combined with other non-carotenoid antioxidants (Beatty S et al. Surv. Opthalmol 2000; 45:115-134; Cait et al. Prog Retin Eye Res 2000; 10:205-211), (Junqueira VB et al. Mol Aspects Med 2004;25:5-16) (Koh HH et al. Experimental Eye Research 2004; 79:21-27; Beatty S et al. Arch Biochem Biophys 2004;430:70-76).

Finally, in the state of the art alpha-tocopherol is known for its beneficial effects as an antioxidant, both from an alimentary perspective and also in the body.

Current inventors have just discovered that the combination of salmon oil enriched with long-chain ω-3 polyunsaturated acids such as EPA and DHA, alpha-tocopherol and supercritical rosemary extract, added to a food product results in an unexpected synergic reaction between the antioxidants and polyunsaturated fatty acids. This is translated into a much greater increase in antioxidant activity than expected. This also helps to maintain levels of the bioactive substances during the manufacture, storage and cooking of enriched food products, with the subsequent beneficial effects for human health on their consumption.

Therefore, the present invention provides a synergic oily composition based on salmon oil enriched with EPA and DHA, alpha-tocopherol and supercritical rosemary extract to use in the preparation of an enriched food product. This composition can also comprise the microalga *Dunaliella salina* that also contains components beneficial for health such as the carotenoids lutein or beta-carotene, for example.

Similarly, the invention also provides food products enriched with this oily mixture with a ratio of ω-3 to ω-6 polyunsaturated fatty acids lower than 5, beneficial in the prevention of diseases such as asthma, cancer or different cardiovascular diseases. This ratio is also maintained during the production, storage and cooking of the food product, due to the synergistic action between these polyunsaturated fatty acids and alpha-tocopherol and the phenolic diterpenes from the supercritical rosemary extract. These enriched food products also conserve the quality characteristics concerning sensorial properties and also safety.

Therefore, the enriched product provided by the invention is beneficial for human health both owing to its stable levels of polyunsaturated fatty acids with an ω-3 to ω-6 ratio lower than 5, and its stable contents of alpha-tocopherol, phenolic diterpenes derived from the supercritical extract of rosemary and, optionally, carotenoids derived from the microalga *Dunaliella salina.*

### OBJECT OF THE INVENTION

Therefore, the aim of the present invention is to provide a synergistic oily mixture based on natural bioactive ingredients to be used in the preparation of an enriched food product that comprises salmon enriched with EPA and DHA, alpha-tocopherol and supercritical rosemary extract.

Another object of the present invention is to provide a food product enriched with this oily mixture based on natural bioactive ingredients.

Finally, another object of the present invention is to provide a method to prepare this enriched food product.

### DETAILLED DESCRIPTION OF THE INVENTION

The present invention provides an oily mixture based on natural bioactive ingredients to be used in the preparation of an enriched food product, characterized in that it comprises salmon oil enriched with EPA and DHA, alpha-tocopherol and supercritical rosemary extract.

In the context of the present application, the term "enriched food product" refers to a food product to whose composition substances have been added that it does not naturally contain or that it comprises in low concentrations.

Similarly, the term "natural bioactive ingredients" refers to compounds of natural origin, with biological activities beneficial to health according to the current state of the art of scientific knowledge.

As mentioned previously, salmon oil enriched in EPA (eicosapentanoic acid) and DHA (docosahexanoic acid) provides omega-3 polyunsaturated fatty acids. These are well known functional ingredients used in the food industry, so their use has a very low risk. The incorporation of ω-3 fatty acids can compensate for the unfavourable lipid profile of the fat from land animals, especially from pigs, since consumption of pork can result in a rise in ω-6 fatty acids. The intervention of these ω-6 fatty acids in redox imbalances at cellular level can lead to an increase in cellular proliferation, such as occurs in cancer; to the triggering of inflammatory processes such as occurs in cardiovascular, autoimmune and neurological diseases; and to deficiencies in neurotransmission causing neurological disorders. Similarly, cellular redox balance affects gene expression in regulators of vital processes and DNA damage generation that produces mutations in key genes.

Hence, the addition of salmon oil enriched with EPA and DHA to food products from land animals for their enrichment, helps to compensate their natural ω-6 / ω-3 imbalance, since, for example, the ω-6 / ω-3 ratio in the animal fat of land animals such as swine and turkey is, on average, higher than 10 (although turkey meat only presents 1% of fat contents compared to the 40% fat contents of pork). In pork fat, the ω-6 / ω-3 ratio is even higher and this ratio in the meat of these animals is much higher than recommended.

However, since the consumption of ω-3 fatty acids can increase oxidative stress, the addition of these fatty acids to food products should be combined with the simultaneous addition of antioxidants such as supercritical rosemary extract or alpha-tocopherol. These antioxidants, as mentioned previously, are known in the state of the art, although, until now, their important synergistic action when combined with salmon oil enriched with EPA and DHA was not known.

Supercritical rosemary extract, as well as helping to reduce the oxidative stress caused by unsaturated fatty acids, has potential protective effects against very serious diseases, as well as being an excellent natural food preservative. For the purpose of this invention, a supercritical rosemary extract sold by Flavex (Austria) can be used, for example, or one prepared by extraction with supercritical CO₂ at pressures ranging from 150 to 250 bars and temperatures between 40 and 70 °C.

On the other hand, alpha-tocopherol presents important benefits as an antioxidant, as mentioned previously. For the purpose of the invention, alpha-tocopherol marketed by Roche can be used, for example.

The synergistic interaction of polyunsaturated fatty acids of enriched salmon oil, alpha-tocopherol and supercritical rosemary extract can achieve a ω-3 /ω-6 ratio lower than 5 and maintain this during the manufacture, storage and cooking of the food product to which the oily mixture is added. This synergistic action results in maintaining the antioxidant activity of alpha-tocopherol and of the supercritical rosemary extract, and in maintaining the alpha-tocopherol contents and the phenolic diterpenic contents from the supercritical rosemary extract in the food product to which the oily mixture of the invention is added.

In one specific embodiment of the invention, the oily mixture comprises:
- 70-99.9% of enriched salmon oil with 10 to 40% of EPA and DHA,
- 0.001-1 % of alpha-tocopherol, and
- 0.1-5% of supercritical rosemary extract,
where these correspond to the percentages by weight with respect to the total weight in the oily mixture

In a preferred embodiment, the oily mixture comprises:
- 80-97% of salmon oil enriched with 10 to 40% of EPA and DHA,
- 0.001-0.1% of alpha-tocopherol, and
- 1-3% of supercritical rosemary extract,
where these correspond to the percentages by weight with respect to the total weight of the oily mixture.

In a more preferred embodiment, the oily mixture comprises:
- 82% of salmon oil enriched with 10 to 40% of EPA and DHA,
- 0.08% of alpha -tocopherol, and
- 1.6% of supercritical rosemary extract,
where these correspond to the percentages by weight with respect to the total weight of the oily mixture.

In another specific embodiment of the invention, the oily mixture also comprises the microalga *Dunaliella salina.* This microalga, as mentioned previously, is one of the ones most used in food products, its toxicity has therefore been well studied and its use does not constitute a health risk. The microalga *Dunaliella salina* has a significant carotenoid content which can enhance the antioxidant action of alpha-tocopherol and of the supercritical rosemary extract, and which also has a preventive action against selected diseases, such as some which affect the sight. Owing to the synergistic action between alpha-tocopherol and the supercritical rosemary extract, this carotenoid content can be maintained in the food product to which the oily mixture of the invention is added.

The object of the invention can be made, for example, with the microalga *Dunaliella salina* marketed by Nature Beta Technologies (NBT) Ltd. (Israel).

In a preferred embodiment of the invention, the oily mixture comprises 0.1-20%, preferably 3-18% and, even more preferably, 16% of the microalga *Dunaliella salina,* where these correspond to percentages by weight with respect to the total weight of the oily mixture.

In another aspect, the invention provides a food product enriched with an oily mixture based on natural bioactive ingredients that contain salmon oil enriched in EPA and DHA, alpha-tocopherol and supercritical extract of rosemary, as described previously.

In a specific embodiment, this food product is enriched with an oily mixture based on natural bioactive ingredients that comprises salmon oil enriched in EPA and DHA, alpha-tocopherol and supercritical rosemary extract, as well as the microalga *Dunaliella salina.*

In a preferred embodiment of the invention, this food product comprises:
- 0.1-20% of salmon oil enriched with 10 to 40% of EPA and DHA.
- 0.00001-1 % of alpha-tocopherol,
- 0.001-5% of supercritical rosemary extract and, optionally,
- 0.01-5% of the microalga *Dunaliella salina,*
where these correspond to the percentages by weight with respect to the total weight of the food product

In a more preferred embodiment of the invention, this enriched food product comprises:
- 1-10% of salmon oil enriched with 10 to 40% of EPA and DHA
- 0.001-0.5% of alpha-tocopherol,
- 0.01-3% of supercritical rosemary extract and, optionally,
- 0.1-3% of the microalga *Dunaliella salina,*
where these correspond to the percentages by weight with respect to the total weight of the food product.

Similarly, in an even preferable embodiment of the invention, this enriched food product comprises:
- 5% of salmon oil enriched with 10 to 40% of EPA and DHA,
- 0.005% of alpha-tocopherol,
- 0.1 % of supercritical extract of rosemary and, optionally,
- 1 % of the microalga *Dunaliella salina,*
where these correspond to the percentages by weight with respect to the total weight of the food product.

The salmon oil is enriched with EPA and DHA in a proportion ranging from 10 to 40% by weight with respect to the total weight of the oil. In a preferred embodiment the salmon oil is enriched with 18% EPA and 12% DHA by weight with respect to the total weight of oil.

For the purpose of the invention, salmon oil can be used enriched with 18% EPA and 12% DHA marketed by Productos Quimicos de Murcia S.A., for example.

The enriched food product of the invention presents a content of polyunsaturated fatty acids with a ratio of ω-6/ω-3 polyunsaturated fatty acids lower than 5, which has important benefits for human health as explained previously. This ratio, thanks to the synergistic interaction of ω-3 fatty acids, alpha-tocopherol and supercritical rosemary extract, is maintained throughout the manufacture, storage and subsequent cooking of the food products enriched in this way.

In one specific embodiment, the enriched food product of the invention is a meat product. Preferably, the enriched food product of the invention is a meat product selected from the group consisting of frankfurter type sausages, cooked ham (boiled ham), cooked turkey breast, cured "chorizo" sausage, cured "salchichon" sausage, cured pork loin and cured ham.

In another aspect, the invention corresponds to a method to prepare this enriched food product that comprises the steps of:
a) preparing an oily mixture based on natural bioactive ingredients, by mixing these natural bioactive ingredients and,
b) incorporating the oily mixture prepared in a) to the food product to be enriched.

In one specific embodiment of this method, the natural bioactive ingredients are combined in a proportion of:
- 70-99.9% of salmon oil enriched with 10 to 40% of EPA and DHA,
- 0.001-1% of alpha-tocopherol,
- 0.1-5% of supercritical rosemary extract and, optionally,
- 0.1-20% of the microalga *Dunaliella salina,*
where these correspond to the percentages by weight with respect to the total weight of the oily mixture.

In a preferred embodiment of the method of the invention, the natural bioactive ingredients are combined in a proportion of:
- 80-97% of salmon oil enriched with 10 to 40% of EPA and DHA,
- 0.001-0.1% of alpha-tocopherol
- 1-3% of supercritical extract of rosemary and, optionally,
- 3-18% of the microalga *Dunaliella salina,*
where these correspond to the percentages by weight with respect to the total weight of the oily mixture.

In an even preferable embodiment of the method of the invention, the natural bioactive ingredients are combined in a proportion of:
- 82% of salmon oil enriched with 10 to 40% of EPA and DHA,
- 0.08% of alpha-tocopherol,
- 1.6% of supercritical extract of rosemary and, optionally,
- 16% of the microalga *Dunaliella salina,*
where these correspond to the percentages by weight with respect to the total weight of the oily mixture.

Hence, to prepare the enriched food products of the invention, suitable amounts of each of the functional ingredients are weighed and then mixed to obtain an oily and slightly coloured product.

If the food product to be enriched corresponds to a meat product such as frankfurter sausages, the oily mixture of the bioactive ingredients is added during the mixing process and production of the meat emulsion. The meat emulsion is then used to fill sausage casings, the sausages are cooked, vacuum packed and refrigerated for a maximum period between 30 and 90 days.

For enriched boiled ham, the oily mixture of bioactive ingredients is injected into raw cuts of ham together with the brine. After this, the cuts are placed in a massage drum to help the mixture of bioactive compounds to spread internally throughout the meat in a uniform way. The cuts are then cooked, vacuum packed and then kept in refrigeration for a maximum time ranging between 30 and 90 days.

To prepare enriched cooked turkey breast, the oily mixture of bioactive ingredients is injected into the raw breast cuts together with the brine. Next, the cuts are placed in a massage drum to help the mixture of bioactive compounds to spread internally throughout the meat in a uniform way. Next, the meat cuts are cooked and vacuum-packed, and stored under refrigeration for a maximum period ranging from 30 to 90 days.

To prepare enriched cured loin, the oily mixture of bioactive ingredients is injected into the cuts of raw pork loin. Next, casings are filled with the meat and cured.

To prepare enriched cured ham, the oily mixture of bioactive ingredients is spread on the surface of raw ham cuts together with the salt. The cuts are then mildly pressed and cured.

To prepare enriched cured chorizo, the oily mixture of bioactive ingredients is combined with the minced meat and spices. Next, sausages are made with the mixture and these are cured.

To prepare enriched cured "salchichon", the oily mixture of bioactive ingredients is combined with the minced meat and the spices. Then, sausages are made with the mixture and these are cured.

Hence, in summary, owing to the addition of the synergistic oily mixture of the invention, the enriched food product presents the following advantages:
1. It has a ω-6 / ω-3 ratio lower than 5, and this fatty acid profile is maintained throughout the manufacturing processes, during its shelf life and also during the cooking processes of these products, such as frying.
2. It does not present a significant increase in oxidation index owing to its incorporation of PUFA, but this remains almost unaltered during its manufacture, storage and cooking.
3. It does not present a significant decrease in antioxidant activity of the antioxidant products added during its manufacture, storage and cooking.
4. It does not present significant changes in alpha-tocopherol contents during its manufacture, storage and cooking.
5. It does not present significant changes in phenolic diterpene contents provided by the supercritical rosemary extract during its manufacture, storage and cooking.
6. It does not present significant alterations in carotenoids provided by the microalga *Dunalliela salina* during its manufacture, storage and cooking.

The following are two examples of enriched food products that encompass all the possible processing operations for meat products:
- **Example 1**. Frankfurter type sausages, demonstrating how to obtain and conserve the described properties in a process including cooking and storage for 60 days refrigerated and under vacuum, and subsequent frying.
- **Example 2.** Cured Iberian chorizo, demonstrating how to obtain and conserve the described properties in a process with 50 days curing.

These examples are given to help understand the invention clearly. The scope of the invention is not limited in any way to these.

### METHODS

### 1. Fatty acid profile

Extraction: different methods were evaluated to extract the lipid fraction present in the samples: a) hexane, b) hexane/methanol, and c) hexane/water (5/1). The methods a) and b) produced interphases that made it difficult to separate the hexane phase. Method c) was the only one tested that enabled the hexane phase to be separated correctly, so this was chosen for the remaining extractions.

Lipid extraction protocol: 5 grams of sample were previously ground to homogenize the sample. Then, 1 g of each sample was placed in a 50 ml falcon vial and 5 ml of milli-Q H₂O were added, followed by 25 ml of hexane. The sample was shaken vigorously with an Ultra Turrax for 1 minute and the supernatant was collected. In some cases, a centrifugation step was required to completely separate the aqueous phase and the hexane phase. This centrifugation was carried out at 3800 rpm for 5 minutes. In order to ensure that most of the fat in the sample had been extracted, a second extraction was done with 25 ml of hexane. In each extract, the hexane was evaporated to constant weight in a rota-vapor at 40°C, and the residue obtained was kept in a vial under nitrogen atmosphere protected from the sunlight.

Extract derivatization protocol: Solutions were prepared of 25 mg/ml (for samples without the salmon oil) and 50 mg/ml (for samples with the salmon oil) concentrations of the extracts in chloroform/methanol 2/1 (v/v). A total of 0.5 ml of these solutions were methylated with NaOH in methanol (0.1M), at 60 °C for 30 min. Next, the derivatization was stopped by adding 0.2 ml of mQ water. Afterwards, the methyl ester fatty acids formed were extracted twice with 1 ml of hexane. In order to remove the residual water from the hexane phase, the fractions were dried with anhydrous sodium sulphate.

Chromatographic method for the lipid analysis: Analyses were performed in a Perkin-Elmer autosystem XL chromatograph, with a BTR-Carbowax column of the following dimensions: L = 30 m; I.D.: 250 µm; phase thickener: 0.25 µm. The chromatographic method used was as follows:
Injector temperature: 220 °C
Furnace temperature programme: 100 °C ---180 °C (at 20 °C/min) - 220 °C (at 15 °C/min) (33 min)
FID detector temperature: 230 °C
Total analysis time: 40 min.
He pressure: 4 bars (4.10⁵ Pa)
Synthetic air pressure: 4 bars (4 .10⁵ Pa)
Hydrogen pressure: 2 bars (2 .10⁵ Pa)
Column head pressure: 12 bars (12 .10⁵ Pa)
He flow: 1 ml/min.
Split Ratio: 20: 1
Injection volume: 1 µl

The retention times of the different methyl ester fatty acids were determined by injecting a solution of 20 mg/ml (in hexane) of PUFA N°1 Marine Source, Supelco (4-7033).

### 2. Oxidation index

This method is based on quantification of the malondialdehyde (MDA) produced as the final compound of lipid oxidation. To measure this compound, it was extracted from the sample using trichloroacetic acid and then quantified by colorimetric reaction with thiobarbituric acid, resulting in the formation of a pink coloured adduct, with a maximum absorbance peak at 531 nm. For the quantification method: 10 g of sample were taken (± 0.005 g) and the weight recorded, 20 ml of 10% trichloroacetic acid were added and the sample was homogenised for 30 seconds at 20000 rpm. Afterwards, this was centrifuged for 30 minutes at 4000 rpm and 10°C. After centrifugation, the sample was filtered and 2 ml of supernatant was collected in a test-tube. To these 2 ml of supernatant, another 2 ml of a solution of thiobarbituric acid were added (TBA, 300 mg/100 ml); the solution was mixed in a vortex, covered with silver foil and placed for 20 minutes in a water-bath with boiling water. It was then left to cool to ambient temperature and the colour formed at 531 nm was measured. In order to measure the colour of the sample itself, the same tests were performed on a blank as were performed on the samples, substituting 2 ml of TBA for 2 ml water.

### 3. Antioxidant activity

Extraction of the added compounds was performed by adding ethanol (20 ml of ethanol per 10 g of sample) and the filtrate obtained after centrifugation was taken to dryness. The dry residue obtained in each case was dissolved in ethanol at a concentration of 15 mg/ml. A total of 0.1 ml of this solution were used to estimate the antioxidant capacity of the different compounds by the β-carotene bleaching test, which produced a concentration of the study compound in the reaction medium of 60 µg/ml. The β-carotene bleaching test estimates the capacity of a substance with a potentially anti-oxidant effect to inhibit β-carotene oxidation, when this is in an emulsion with linoleic acid in pro-oxidant conditions.

### 4. Analysis of tocopherols

### Preparation of samples

To quantify the tocopherol content of the salmon oil added to the mixtures, 20 µl of oil were directly injected in HPLC. From each sample, 10 g were taken and mixed with 20 ml of ethanol. This was homogenized in the ultraturrax for 1 minute and centrifuged. The supernatant was passed through a filter and concentrated to dryness in a rotavapor. Then 2 ml of ethanol were added. The concentrates were passed through a filter and injected in HPLC for analysis using a reverse phase column (Nova-Pak C18 60A 4µm 3.9 x 150 mm, Waters) and were developed at a flow of 1 ml/min following an isocratic method of a mixture of 97% methanol in 1 % acetic acid (v/v) for 20 min. Peaks were detected with a photodiode detector to identify peaks by retention time and their spectrum in relation to the standards mentioned and were quantified at a maximum wavelength for most of the compounds (295 nm).

To quantify the areas detected, calibration curves were developed using tocopherol standards to quantify the peaks corresponding to the samples.

### 5. Antioxidants of rosemary extract

Extraction method: A total of 10 grams of each of the samples were weighed and 20 ml of acetone were added to each. After homogenization for 1 minute in the ultraturrax, they were left to rest for 2 hours to facilitate phase separation. Next, they were centrifuged at 3500 r.p.m for 30 minutes. The supernatant was filtered through filter paper and then evaporated in the rotavapor.

Chromatographic method: Analyses were carried out in a HPLC system with a NovaPack C₁₈ column of 150 mm length, 4.6 mm internal diameter and a particle size of 3.5 µm. The mobile phase used in the separation consisted in a mixture of solvents A (acetonitrile with 1% acetic acid) and B (water with 1 % acetic acid). The composition of the mobile phase varied along a 30 minute gradient, starting with 50% of B for 5 minutes, 30% of B at 15 minutes and reaching 0% of B at 30 min. The flow was maintained during the entire separation at 0.7 ml/min. Compounds were detected with a diode beam detector in a wavelength ranging from 200 to 450 nm. The detection slit was established at 4 nm and the sampling interval at 200 ms. The wavelength selected for the detection of compounds was 230 nm. The equipment was furnished with a 20 µl injector.

### 6. Carotenoid profile

Extraction of carotenoids from the microalgae: Extracts of 0.05 g/ml of *Spirulina* and *Dunaliella* were prepared in petroleum ether: acetone (1:1) to compare the carotenoid concentration of both algae. An extract was prepared of 0.005 g/ml of *Dunaliella* (corresponding to 1 % added to the samples) in terc-butyl methyl ether to quantify the loss of carotenoids produced in the extraction of carotenoids, because only one extraction from the samples was performed. A second extraction was done to corroborate experimental data with data in the literature.

Extraction of carotenoids from the samples. A total of 5 g of each sample were weighed and shredded for 1 minute, with 5 s pauses, in a domestic food processor. A total of 5 g of the shredded mixture was mixed with 10 ml of terc-butyl methyl ether. The mixture was homogenised in an Ultraturrax for 1 min and left to rest until the two phases separated (in the dark). The supernatant (20 µl) was immediately injected into the HPLC for analysis.

HPLC analysis: The samples and standards were injected in a HPLC using a reverse phase column (Microsorb C18, 250 x 4.6 mm of Varian) and were developed at a flow of 1 ml/min following a gradient starting with 50% of mixture B, which increased in 14 min to 100% B and remained constant to the end of development at 53 minutes. The mixtures of solvents used corresponded to: mixture A: dichloromethane: methanol: acetonitrile: water (0:60:5:35) and mixture B: dichloromethane: methanol: acetonitrile: water (25:28:42.5:4.5). Peak detection was performed using a photodiode detector to identify the peaks from their retention times and their spectrum in relation to the standards mentioned, and were quantified at a maximum wavelength for most of the compounds (450 nm). To quantify the areas detected, calibration curves are developed using lutein to quantify the lutein peaks of the samples. The peaks of β-carotene and 9-cis-β-carotene are quantified with the straight line obtained from the β-carotene curve, owing to the similarity of their spectrum.

### EXAMPLE 1. Frankfurter type sausages.

### PREPARATION

After obtaining the standard meat emulsion to manufacture frankfurter type sausages, the following amounts of the ingredients of the oily mixture are added, per kg of meat paste:
- 50 grams of salmon oil, deodorized and enriched with 18% EPA and 12% DHA.
- 1 gram of supercritical rosemary extract
- 0.05 grams of alpha-tocopherol
- 10 grams of *Dunaliella salina*

The oily mixture is added to the meat paste in a mixer in order to obtain an emulsion with a homogeneous distribution of the oily mixture ingredients. Afterwards, this was made into sausages and cooked at 70 °C for 60 minutes. Next, the sausages were vacuum-packed and refrigerated at 5 °C for 90 days. Frying was done at 180 °C for three minutes.

### RESULTS

The following Table 1.1. shows the lipid profile for the sausages determined after the processing operations and at different storage times.

**Table 1.1 Molar percentage of fatty acids**

| | Control Paste | Paste not in sausages | Cooked sausage | Sausage after 21 days storage | Sausages after 60 days storage | Fried sausage |
|---|---|---|---|---|---|---|
| **Myristic (C14:0)** | 1.4 | 3.0 | 3.0 | 2.1 | 2.1 | 2.9 |
| **Palmitic (C16:0)** | 25.0 | 23.0 | 23.9 | 23.7 | 22.9 | 23.5 |
| **Palmitoleic (C16:1)** | 2.3 | 4.4 | 4.1 | 4.0 | 3.9 | 4.2 |
| **Stearic (C18:0)** | 12.4 | 10.7 | 11.4 | 11.9 | 11.3 | 11.0 |
| **Oleic (C18:1)** | 40.7 | 35.0 | 36.1 | 36.9 | 35.3 | 36.0 |
| **Linoleic (C18:2) n-6** | 14.7 | 12.3 | 12.5 | 12.4 | 12.1 | 12.6 |
| **Linolenic (C18:3) n-3** | 0.8 | 0.8 | 0.8 | 0.8 | 0.7 | 0.8 |
| Stearidonic (C18:4) n-3 | 0.0 | 0.6 | 0.5 | 0.4 | 0.4 | 0.5 |
| **C20:1** | 0.6 | 0.8 | 0.7 | 0.9 | 0.7 | 0.7 |
| **EPA (C20:5) n-3** | 0.0 | 4.5 | 3.3 | 3.2 | 3.2 | 3.5 |
| **DPA (C22:5) n-3** | 0.0 | 0.4 | 0.3 | 0.3 | 0.3 | 0.3 |
| **DHA (C22:6) n-3** | 0.0 | 2.6 | 1.5 | 1.5 | 2.0 | 2.0 |
| | | | | | | |
| **saturated** | 38.8 | 36.7 | 38.3 | 37.6 | 36.2 | 37.4 |
| **monounsaturated** | 43.7 | 40.2 | 41.0 | 41.8 | 40.0 | 41.0 |
| **n-6** | 14.7 | 12.3 | 12.5 | 12.4 | 12.1 | 12.6 |
| **n-3** | 0.8 | 8.9 | 6.3 | 6.2 | 6.6 | 7.1 |
| n-6/n-3 | 17.9 | 1.4 | 2.0 | 2.0 | 1.8 | 1.8 |

From the data from Table 1.1., it can be deduced first of all that with the addition of 50g/kg of salmon oil enriched in EPA and DHA, the ω-6 / ω-3 ratio is reduced from 17.9 to a value below 2, which is maintained during the entire process of production, storage and cooking. Moreover, the lipid profile also remains stable.

It is essential to maintain the antioxidant activity of the oily mixture during processing and storage to achieve the intended objectives. Moreover, the antioxidant activity contributes to maintaining the lipid profile stable since the PUFA are oxidizable.
Table 1.2. shows the oxidation index data for the sausages.

**Table 1.2. Oxidation index**

| | Control Paste | Paste not in sausages | Cooked sausage | Sausage at 21 days of storage | Sausage at 60 days storage | Fried sausage |
|---|---|---|---|---|---|---|
| mg MDA/Kg | 0.10 | 0.27 | 0.32 | 0.29 | 0.32 | 0.35 |

Even with the addition of an appreciable amount of PUFA, the oxidation index remained low during the entire processing period and storage. This result coincides with others presented and confirms maintenance of the lipid profile of PUFA, and therefore the ω-3 6 / ω-3 ratio, and the antioxidant activity of the oily mixture ingredients.

Table 1.3. presents the results of antioxidant activity analysis for the sausages.

**Table 1.3. Antioxidant activity**

| | Control paste | Paste not in sausages | Cooked sausage | Sausage after 21 days storage | Sausage after 60 days storage | Fried sausage |
|---|---|---|---|---|---|---|
| Antioxidant activity | 19.23% | 68.73% | 57.48% | 56.58% | 51.80% | 63.66% |

Addition of the oily mixture increases the antioxidant activity by a factor of 3.4 fold the value determined in sausages before the process. This antioxidant activity is reduced slightly during the processing and storage, but during the shelf-life never falls below 2.5 fold the value obtained for the product without the oily mixture.

The rise in antioxidant activity after frying can be due to the effect of adsorption of the used oil (virgin olive oil).

Table 1.4. shows the results of the alpha-tocopherol analysis in sausages. The presence of alpha-tocopherol in the sausages at the end of processing and storage is another indicator of the high antioxidant activity of the oily mixture.

Indeed, in a parallel experiment in which only salmon oil plus alpha-tocopherol was added to the sausages, alpha-tocopherol was not even detected before cooking. At this moment, the antioxidant activity was 32.92%, in other words, less than half that obtained when the complete oily mixture was added. This demonstrates the **synergy between alpha-tocopherol and the supercritical rosemary extract**.

**Table 1.4. Concentration of alpha-tocopherol**

| | Control paste | Paste not in sausages | Cooked sausage | Sausage at 21 days storage | Sausage at 60 days storage | Fried sausage |
|---|---|---|---|---|---|---|
| µg/g alpha-tocopherol | 0.0 | 30.0 | 14.5 | 11.5 | 10.2 | 8.7 |

The presence of components of the supercritical rosemary extract is an indicator of its permanence in the sausages during the process. Table 1.5 shows the results of the carnosic acid analysis, the most active antioxidant component of the supercritical rosemary extract and, also, the most labile.

**Table 1.5. Concentration of carnosic acid**

| | Control paste | Paste not in sausages | Cooked sausages | Sausage at 21 days after storage | Sausage at 60 days after storage | Fried sausage |
|---|---|---|---|---|---|---|
| mg/10g carnosic acid | 0.0 | 224.9 | 198.5 | 167.6 | 141.9 | 140.2 |

Although the amount of carnosic acid present in the sausages was found to diminish as the process advanced and during storage, the presence of significant amounts of this compound at the end of the process, even after cooking, was demonstrated.

Table 1.6. gives the results of the carotenoid analysis in sausages. These compounds are derived from the microalga *Dunaliella salina.*

**Table 1.6. Carotenoid concentration**

| | Control paste | Paste not in sausages | Cooked sausage | Sausage after 21 days storage | Sausage after 60 days storage | Fried sausage |
|---|---|---|---|---|---|---|
| mg/g lutein | 0.001 | 0.01 | 0.01 | 0.02 | 0.01 | 0.02 |
| mg/g de betacarotene | 0.004 | 0.42 | 0.37 | 0.37 | 0.28 | 0.41 |

Although some oscillations can been observed, it is found that the carotenoids remained stable throughout the process. The increase during the final steps could be due to the release of these compounds inside the microalga cells.

### CONCLUSION

Incorporation of the oily mixture into the frankfurter sausages confers these an antioxidant activity, a contents of natural antioxidants and a ω-6 / ω-3 ratio >5 that remain stable throughout the production process, storage and cooking.

### EXAMPLE 2. Cured Iberian chorizo.

### PREPARATION

To the standard ingredients of traditional Iberian chorizo, the following quantities of ingredients of an oily mixture are added per kg of meat paste:
- 50 grams of salmon oil, deodorized and supplemented with 18% EPA and 12% DHA
- 1 gram of supercritical rosemary extract
- 0.05 grams of alpha-tocopherol
- 10 grams of *Dunaliella salina*

Next, the mixture is mixed in an industrial mixer under vacuum, introduced into casings and cured for up to 50 days.

### RESULTS

The following Table 2.1. shows the lipid profile of cured chorizo determined after processing operations and at different storage times.

**Table 2.1. Molar percentage of fatty acids determined**

| | **Control mixture** | **Chorizo** | **Chorizo 25 days curing** | **Chorizo 50 days curing** |
|---|---|---|---|---|
| **Myristic (C14:0)** | 1.3 | 3.9 | 3.0 | 2.2 |
| **Palmitic (C16:0)** | 27.5 | 25.2 | 26.5 | 26.5 |
| **Palmitoleic (C16:1)** | 2.6 | 5.6 | 4.8 | 4.5 |
| **Stearic (C18:0)** | 12.7 | 10.8 | 11.7 | 12.0 |
| **Ocleic (C18:1)** | 46.6 | 36.5 | 40.1 | 40.6 |
| L**inoleic (C18:2) n-6** | 6.1 | 5.1 | 5.2 | 6.2 |
| **Linolenic (C18:3) n-3** | 0.4 | 0.5 | 0.3 | 0.5 |
| **Stearidonic (C18:4) n-3** | 0.0 | 0.7 | 0.3 | 0.4 |
| **C20:1** | 0.7 | 0.8 | 0.8 | 0.8 |
| **EPA (C20:5) n-3** | 0.0 | 5.3 | 3.0 | 2.3 |
| D**PA (C22:5) n-3** | 0.0 | 0.4 | 0.3 | 0.3 |
| **DHA (C22:6) n-3** | 0.0 | 3.2 | 2.0 | 1.5 |
| | | | | |
| **Saturated** | 41.6 | 39.9 | 41.2 | 40.8 |
| **Monounsaturated** | 49.9 | 42.9 | 45.6 | 46.0 |
| **n-6** | 6.1 | 5.1 | 5.2 | 6.2 |
| **n-3** | 0.4 | 10.1 | 5.9 | 5.0 |
| **n-6/n-3** | 14.2 | 0.5 | 0.9 | 1.2 |

From the data in Table 2.1. it can first be deduced that with the addition of 50g/kg of salmon oil enriched with EPA and DHA, the ω-6 / ω-3 ratio is reduced from 14.2 to a value close to 1, and this is maintained during the entire production process, including a curing process of 50 days.

Regarding the determinations of antioxidant activity, alpha-tocopherol, carnosic acid, carotenoids and oxidation index, the results are analogous to those presented for the sausages.

### CONCLUSION

Incorporation of the oily mixture into cured Iberian chorizo confers it an antioxidant activity, contents of natural antioxidants and an ω-6 / ω-3 ratio >5 that remain stable throughout the entire process, including the 50 days curing time.

## Claims

1. An oily mixture based on natural bioactive ingredients to be used in the preparation of an enriched food product, **characterized in that** it comprises a salmon oil enriched in EPA and DHA, alpha-tocopherol and a supercritical rosemary extract.

2. An oily mixture according to claim 1, **characterized in that** it comprises:
- 70-99.9% of salmon oil enriched with 10 to 40% of EPA and DHA,
- 0.001-1% of alpha-tocopherol, and
- 0.1-5% of supercritical rosemary extract,
where these correspond to percentages by weight with respect to the total weight of the oily mixture.

3. Oily mixture according to claim 2, **characterized in that** it comprises:
- 80-97% of salmon oil enriched with 10 to 40% of EPA and DHA,
- 0.001-0.1% of alpha-tocopherol, and
- 1-3% of supercritical rosemary extract,
where these correspond to percentages by weight with respect to the total weight of the oily mixture.

4. Oily mixture according to claim 3, **characterized in that** it comprises:
- 82% of salmon oil enriched with 10 to 40% of EPA and DHA,
- 0.08% of alpha-tocopherol, and
- 1.6% of supercritical rosemary extract,
where these correspond to percentages by weight with respect to the total weight of the oily mixture.

5. Oily mixture according to any of claims 1 to 4, **characterized in that** it also comprises the microalga *Dunaliella salina.*

6. Oily mixture according to claim 5, **characterized in that** it comprises 0.1-20%, preferably 3-18% and, even more preferably, 16% of microalga *Dunaliella salina,* where these correspond to percentages by weight with respect to the total weight of the oily mixture.

7. A food product enriched with an oily mixture based on natural bioactive ingredients that comprises salmon oil enriched with EPA and DHA, alpha-tocopherol and supercritical rosemary extract.

8. Food product enriched according to claim 7, **characterized in that** the oily mixture based on natural bioactive ingredients also comprises the microalga *Dunaliella salina.*

9. Food product enriched according to any of claims 7 to 8, **characterized in that** it comprises:
- 0.1-20% of salmon oil enriched with 10 to 40% of EPA and DHA
- 0.00001-1% of alpha-tocopherol,
- 0.001-5% of supercritical rosemary extract and, optionally,
- 0.01-5% of the microalga *Dunaliella salina,*
where these correspond to percentages by weight with respect to the total weight of the oily mixture.

10. Food product enriched according to claim 9, **characterized in that** it comprises:
- 1-10% of salmon oil enriched with 10 to 40% of EPA and DHA
- 0.001-0.5% of alpha-tocopherol,
- 0.01-3% of supercritical rosemary extract and, optionally,
- 0.1-3% of the microalga *Dunaliella salina,*
where these correspond to percentages by weight with respect to the total weight of the food product.

11. Food product enriched according to claim 10, **characterized in that** it comprises:
- 5% of salmon oil enriched with 10 to 40% of EPA and DHA
- 0.005% of alpha-tocopherol,
- 0.1% of supercritical rosemary extract and, optionally,
- 1 % of the microalga *Dunaliella salina,*
where these correspond to percentages by weight with respect to the total weight of the food product.

12. Food product according to any of claims 7 to 11, **characterized in that** it presents a polyunsaturated fatty acid contents with a ratio of w-6/w-3 polyunsaturated fatty acids lower than 5.

13. Food product enriched according to claim 12, **characterized in that** it is a meat product.

14. Food product enriched according to claim 13, **characterized in that** the meat product is selected from the group consisting of frankfurter sausages, cooked ham, cooked turkey breast, cured chorizo, cured salchichon, cured pork loin and cured ham.

15. A method to prepare a food product enriched according to any of claims 7-14, **characterized in that** it comprises the steps of:
a) preparing an oily mixture based on natural bioactive ingredients by mixing these natural bioactive ingredients and,
b) incorporating the oily mixture prepared in a) to the food product to be enriched.

16. Method according to claim 15, **characterized in that** the natural bioactive ingredients are combined in a proportion of:
- 70-99.9% of salmon oil enriched with 10 to 40% of EPA and DHA,
- 0.001-1% of alpha-tocopherol,
- 0.1-5% of supercritical rosemary extract and, optionally,
- 0.1-20% of the microalga *Dunaliella salina,*
where these correspond to percentages by weight with respect to the total weight of the oily mixture.

17. Method according to claim 16, **characterized in that** the natural bioactive ingredients are combined in a proportion of:
- 80-97% of salmon oil enriched with 10 to 40% of EPA and DHA,
- 0.001-0.1% of alpha-tocopherol,
- 1-3% of supercritical rosemary extract and, optionally,
- 3-18% of the microalga *Dunaliella salina,*
where these correspond to percentages by weight with respect to the total weight of the oily mixture.

18. Method according to claim 17, **characterized in that** the natural bioactive ingredients are mixed in a proportion of:
- 82% of salmon oil enriched with 10 to 40% of EPA and DHA,
- 0.08% of alpha-tocopherol,
- 1.6% of supercritical rosemary extract and, optionally,
- 16% of the microalga *Dunaliella salina,*
where these correspond to percentages by weight with respect to the total weight of the oily mixture.

## Patentansprüche

1. Öliges Gemisch auf Basis natürlicher biologisch aktiver Zutaten für die Verwendung bei der Zubereitung eines angereicherten Nahrungsmittelproduktes, **dadurch gekennzeichnet, dass** es ein mit EPA und DHA angereichertes Lachsöl, alpha-Tocopherol und einen überkritischen Rosmarinextrakt aufweist.

2. Ölgemisch nach Anspruch 1, **dadurch gekennzeichnet, dass** es Folgendes aufweist:
- 70-99,9 % Lachsöl, das mit 10 bis 40 % EPA und DHA angereichert ist,
- 0,001-1 % alpha-Tocopherol und
- 0,1-5 % überkritischen Rosmarinextrakt,
wobei diese Angaben Prozentanteilen nach Gewicht in Bezug auf das Gesamtgewicht des öligen Gemisches entsprechen.

3. Ölgemisch nach Anspruch 2, **dadurch gekennzeichnet, dass** es Folgendes aufweist:
- 80-97 % Lachsöl, das mit 10 bis 40 % EPA und DHA angereichert ist,
- 0,001-0,1 % alpha-Tocopherol und
- 1-3 % überkritischen Rosmarinextrakt,
wobei diese Angaben Prozentanteilen nach Gewicht in Bezug auf das Gesamtgewicht des öligen Gemisches entsprechen.

4. Ölgemisch nach Anspruch 3, **dadurch gekennzeichnet, dass** es Folgendes aufweist:
- 82 % Lachsöl, das mit 10 bis 40 % EPA und DHA angereichert ist,
- 0,08 % alpha-Tocopherol und
- 1,6 % überkritischen Rosmarinextrakt,
wobei diese Angaben Prozentanteilen nach Gewicht in Bezug auf das Gesamtgewicht des öligen Gemisches entsprechen.

5. Öliges Gemisch nach einem der Ansprüche 1 bis 4, **dadurch gekennzeichnet, dass** es auch die Mikroalge *Dunaliella salina* aufweist.

6. Öliges Gemisch nach Anspruch 5, **dadurch gekennzeichnet, dass** es 0,1-20 %, vorzugsweise 3-18 % und insbesondere 16 % der Mikroalge *Dunaliella salina* aufweist, wobei diese Angaben Prozentanteilen nach Gewicht in Bezug auf das Gesamtgewicht des öligen Gemisches entsprechen.

7. Nahrungsmittelprodukt, das mit einem öligen Gemisch auf Basis natürlicher biologisch aktiver Zutaten angereichert ist, das mit EPA und DHA angereichertes Lachsöl, alpha-Tocopherol und einen überkritischen Rosmarinextrakt aufweist.

8. Nahrungsmittelprodukt nach Anspruch 7, **dadurch gekennzeichnet, dass** das ölige Gemisch auf Basis natürlicher biologisch aktiver Zutaten auch die Mikroalge *Dunaliella salina* aufweist.

9. Angereichertes Nahrungsmittelprodukt nach einem der Ansprüche 7 bis 8, **dadurch gekennzeichnet, dass** es Folgendes aufweist:
- 0,1-20 % Lachsöl, das mit 10 bis 40 % EPA und DHA angereichert ist,
- 0,00001-1 % alpha-Tocopherol,
- 0,001-5 % überkritischen Rosmarinextrakt und wahlweise
- 0,01-5 % der Mikroalge *Dunaliella salina,*
wobei diese Angaben Prozentanteilen nach Gewicht in Bezug auf das Gesamtgewicht des öligen Gemisches entsprechen.

10. Angereichertes Nahrungsmittelprodukt nach Anspruch 9, **dadurch gekennzeichnet, dass** es Folgendes aufweist:
- 1-10 % Lachsöl, das mit 10 bis 40 % EPA und DHA angereichert ist,
- 0,001-0,5 % alpha-Tocopherol,
- 0,01-3 % überkritischen Rosmarinextrakt und wahlweise
- 0,1-3 % der Mikroalge *Dunaliella salina,*
wobei diese Angaben Prozentanteilen nach Gewicht in Bezug auf das Gesamtgewicht des öligen Gemisches entsprechen.

11. Angereichertes Nahrungsmittelprodukt nach Anspruch 10, **dadurch gekennzeichnet, dass** es Folgendes aufweist:
- 5 % Lachsöl, das mit 10 bis 40 % EPA und DHA angereichert ist,
- 0,005 % alpha-Tocopherol,
- 0,01 % überkritischen Rosmarinextrakt und wahlweise
- 1 % der Mikroalge *Dunaliella salina,*
wobei diese Angaben Prozentanteilen nach Gewicht in Bezug auf das Gesamtgewicht des öligen Gemisches entsprechen.

12. Nahrungsmittelprodukt nach einem der Ansprüche 7 bis 11, **dadurch gekennzeichnet, dass** es einen Gehalt an mehrfach ungesättigten Fettsäuren mit einem Verhältnis von mehrfach ungesättigten ω-6-/ω-3-Fettsäuren unter 5 aufweist.

13. Angereichertes Nahrungsmittelprodukt nach Anspruch 12, **dadurch gekennzeichnet, dass** es ein Fleischprodukt ist.

14. Angereichertes Nahrungsmittelprodukt nach Anspruch 13, **dadurch gekennzeichnet, dass** das Fleischprodukt aus der Gruppe ausgewählt ist, die aus Frankfurter Würstchen, gekochtem Schinken, gekochter Truthahnbrust, geräucherter Chorizo, geräucherte Salchichón, geräucherter Schweinelende und geräuchertem Schinken besteht.

15. Verfahren zum Zubereiten eines angereicherten Nahrungsmittelproduktes nach einem der Ansprüche 7-14, **dadurch gekennzeichnet, dass** es folgende Schritte aufweist:
a) Zubereiten eines öligen Gemisch auf Basis natürlicher biologisch aktiver Zutaten durch Mischen dieser natürlichen biologisch aktiven Zutaten und
b) Einbinden des in a) zubereiteten öligen Gemisches in das anzureichernde Nahrungsmittelprodukt.

16. Verfahren nach Anspruch 15, **dadurch gekennzeichnet, dass** die natürlichen biologisch aktiven Zutaten in einem Anteil von:
- 70-99,9 % Lachsöl, das mit 10 bis 40 % EPA und DHA angereichert ist,
- 0,001-1 % alpha-Tocopherol und
- 0,1-5 % überkritischem Rosmarinextrakt und wahlweise
- 0,1-20 % der Mikroalge *Dunaliella salina,*
kombiniert sind, wobei diese Angaben Prozentanteilen nach Gewicht in Bezug auf das Gesamtgewicht des öligen Gemisches entsprechen.

17. Verfahren nach Anspruch 16, **dadurch gekennzeichnet, dass** die natürlichen biologisch aktiven Zutaten in einem Anteil von:
- 80-97 % Lachsöl, das mit 10 bis 40 % EPA und DHA angereichert ist,
- 0,001-0,1 % alpha-Tocopherol und
- 1-3 % überkritischem Rosmarinextrakt und wahlweise
- 3-18 % der Mikroalge *Dunaliella salina,*
kombiniert sind, wobei diese Angaben Prozentanteilen nach Gewicht in Bezug auf das Gesamtgewicht des öligen Gemisches entsprechen.

18. Verfahren nach Anspruch 17, **dadurch gekennzeichnet, dass** die natürlichen biologisch aktiven Zutaten in einem Anteil von:
- 82 % Lachsöl, das mit 10 bis 40 % EPA und DHA angereichert ist,
- 0,08 % alpha-Tocopherol und
- 1,6 % überkritischem Rosmarinextrakt und wahlweise
- 16 % der Mikroalge *Dunaliella salina,*
gemischt sind, wobei diese Angaben Prozentanteilen nach Gewicht in Bezug auf das Gesamtgewicht des öligen Gemisches entsprechen.

## Revendications

1. Mélange huileux basé sur des ingrédients naturels bioactifs à utiliser dans la préparation d'un produit alimentaire enrichi, **caractérisé en ce qu'**il comprend une huile de saumon enrichie en EPA et DHA, alpha-tocophérol et un extrait supercritique de romarin.

2. Mélange huileux selon la revendication 1, **caractérisé en ce qu'**il comprend:
- 70-99,9% d'huile de saumon enrichie de 10 a 40% d'EPA et DHA
- 0,001-1% d'alpha-tocophérol, et
- 0,1-5% d'extrait supercritique de romarin,
où ceux-ci correspondent à des pourcentages en poids par rapport au poids total du mélange huileux.

3. Mélange huileux selon la revendication 2, **caractérisé en ce qu'**il comprend:
- 80-97% d'huile de saumon enrichie de 10 a 40% d'EPA et DHA
- 0,001-1% d'alpha-tocophérol, et
- 1-3% d'extrait supercritique de romarin,
où ceux-ci correspondent à des pourcentages en poids par rapport au poids total du mélange huileux.

4. Mélange huileux selon la revendication 3, **caractérisé en ce qu'**il comprend:
- 82% d'huile de saumon enrichie de 10 a 40% d'EPA et DHA
- 0,08% d'alpha-tocophérol, et
- 1,6% d'extrait supercritique de romarin,
où ceux-ci correspondent à des pourcentages en poids par rapport au poids total du mélange huileux.

5. Mélange huileux selon l'une quelconque des revendications 1 à 4, **caractérisé en ce qu'**il comprend en outre la microalgue *Dunaliella salina.*

6. Mélange huileux selon la revendication 5, **caractérisé en ce qu'**il comprend 0,1-0,1-20%, de préférence 3-18% et encore mieux, 16% de la microalgue *Duanliella salina,* où ceux-ci correspondent à des pourcentages en poids par rapport au poids total du mélange huileux.

7. Produit alimentaire enrichi d'un mélange huileux basé sur des ingrédients naturels bioactifs qui comprend une huile de saumon enrichie en EPA et DHA, alpha-tocophérol et un extrait supercritique de romarin.

8. Produit alimentaire enrichi selon la revendication 7, **caractérisé en ce que** le mélange huileux basé sur des ingrédients naturels bioactifs comprend en outre, la microalgue *Duanliella salina.*

9. Produit alimentaire selon l'une quelconque des revendications 7 a 8, **caractérisé en ce qu'**il comprend:
- 0,1-20% d'huile de saumon enrichie de 10 a 40% d'EPA et DHA
- 0,00001-1% d'alpha-tocophérol,
- 0,001-5% d'extrait supercritique de romarin, et optionnellement,
- 0,01-5% de la microalgue *Dunalilella salina,*
où ceux-ci correspondent à des pourcentages en poids par rapport au poids total du mélange huileux.

10. Produit alimentaire selon la revendication 9, **caractérisé en ce qu'**il comprend:
- 1-10% d'huile de saumon enrichie de 10 a 40% d'EPA et DHA
- 0,001-0,5% d'alpha-tocophérol,
- 0,01-3% d'extrait supercritique de romarin, et optionnellement,
- 0,1-3% de la microalgue *Dunalilella salina,*
où ceux-ci correspondent à des pourcentages en poids par rapport au poids total du produit alimentaire.

11. Produit alimentaire selon la revendication 10, **caractérisé en ce qu'**il comprend:
- 5% d'huile de saumon enrichie de 10 a 40% d'EPA et DHA
- 0,005% d'alpha-tocophérol,
- 0,1-5% d'extrait supercritique de romarin, et optionnellement,
- 1 % de la microalgue *Dunalilella salina,*
où ceux-ci correspondent à des pourcentages en poids par rapport au poids total du produit alimentaire.

12. Produit alimentaire selon l'une quelconque des revendications 7 a 11, **caractérisé en ce qu'**il présente un contenu en acides gras polyinsaturés ayant un rapport d'acides gras polyinsaturés ω-6/ω-3 inférieur à 5.

13. Produit alimentaire selon la revendication 12, **caractérisé en ce qu'**il s'agit d'un produit carné.

14. Produit alimentaire selon la revendication 13, **caractérisé en ce que** le produit carné est choisi parmi le groupe constitué de saucisses de Francfort, jambon cuir, poitrine de dinde cuite, chorizo sec, saucisson sec, filet de porc sec y jambon sec.

15. Procédé de préparation d'un produit alimentaire enrichi selon l'une quelconque des revendications 7-14, **caractérisé en ce qu'**il comprend les étapes de:
a) préparation d'un mélange huileux basé sur des ingrédients naturels bioactifs, et
b) incorporation du mélange huileux préparé lors de l'étape a) au produit alimentaire à enrichir.

16. Procédé selon la revendication 15, **caractérisé en ce que** les ingrédients naturels bioactifs sont combinés dans une proportion de:
- 70-99,99% d'huile de saumon enrichie de 10 a 40% d'EPA et DHA
- 0,001-1% d'alpha-tocophérol,
- 0,1-5% d'extrait supercritique de romarin, et optionnellement,
- 0,1-20% de la microalgue *Dunalilella salina,*
où ceux-ci correspondent à des pourcentages en poids par rapport au poids total du mélange huileux.

17. Procédé selon la revendication 16, **caractérisé en ce que** les ingrédients naturels bioactifs sont combinés dans une proportion de:
- 80-97% d'huile de saumon enrichie de 10 a 40% d'EPA et DHA
- 0,001-0,1% d'alpha-tocophérol,
- 1-3% d'extrait supercritique de romarin, et optionnellement,
- 3-18% de la microalgue *Dunalilella salina,*
où ceux-ci correspondent à des pourcentages en poids par rapport au poids total du mélange huileux.

18. Procédé selon la revendication 17, **caractérisé en ce que** les ingrédients naturels bioactifs sont combinés dans une proportion de:
- 82% d'huile de saumon enrichie de 10 a 40% d'EPA et DHA
- 0,08% d'alpha-tocophérol,
- 1,6% d'extrait supercritique de romarin, et optionnellement,
- 16% de la microalgue *Dunalilella salina,*
où ceux-ci correspondent à des pourcentages en poids par rapport au poids total du mélange huileux.
